(19)

Europäisches Patentamt

European Patent Office

Office européen des brevets

(11) **EP 4 781 897 A1**

(12) **EUROPEAN PATENT APPLICATION**

(43) Date of publication:
29.07.2026 Bulletin 2026/31

(21) Application number: 25222900.0

(22) Date of filing: 12.12.2025

(51) International Patent Classification (IPC):
*A61B 5/00* (2006.01) *A61B 5/0507* (2021.01)

(52) Cooperative Patent Classification (CPC):
A61B 5/4812; A61B 5/0507; A61B 5/4815;
A61B 5/7264; G01S 13/88

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 23.01.2025 KR 20250010059

(71) Applicant: bitsensing Inc
Seongnam-si, Gyeonggi-do 13105 (KR)

(72) Inventors:
• KIM, Ji Eun
06800 Seoul (KR)
• Noh, Hea Min
05825 Seoul (KR)
• CHOE, Sun Taag
16413 Suwon-si, Gyeonggi-do (KR)

(74) Representative: BCKIP Part mbB
MK1
Landsbergerstraße 98, 3.Stock
80339 München (DE)

(54) **DEVICE AND METHOD FOR SLEEP STAGE CLASSIFICATION**

(57) A sleep stage classification device using radar signals according to embodiments of the present disclosure includes: a transceiver that transmits a radar signal toward a subject and receives a radar signal reflected from the subject; and a sleep stage information generation unit that generates sleep stage information of the subject by inputting the radar signal reflected from the subject into a sleep stage classification model. The sleep stage classification model includes: a preprocessing unit that preprocesses the input radar signal; and a sleep stage classification unit that classifies sleep stages of the subject based on the preprocessed radar signal.

## FIG. 1

EP 4 781 897 A1

## Description

### CROSS-REFERENCE TO RELATED APPLICATION

This application claims the benefit of Korean Patent Application No. 10-2025-0010059 filed on Jan. 23, 2025, with the Korean Intellectual Property Office.

### TECHNICAL FIELD

The present disclosure relates to a device and method for classifying sleep stages during the sleep of a subject using radar signals.

### BACKGROUND

In modern society, sleep disorders are a common problem experienced by many people, with more than 20% of the global population suffering from a decline in sleep quality. Due to this widespread issue, interest in technologies aimed at improving and accurately measuring sleep quality has been increasing. Conventional sleep monitoring technologies help identify and analyze a user's sleep state, but they present several limitations and inconveniences. To evaluate sleep quality, it is crucial to obtain information on each sleep stage. This is because not only the duration of sleep but also the ratios and patterns of deep sleep, REM sleep, and wakefulness are closely related to overall sleep quality. This information can assist in diagnosing the causes of sleep disorders, establishing effective treatment plans, improving overall health, and facilitating recovery from fatigue.

Conventional sleep monitoring methods generally involve directly attaching sensors or devices to the user's body. For example, sensors are commonly attached to the arm or chest to detect physiological data, such as body movement, respiration, and heart rate. Although this method can measure physiological data with relatively high accuracy, the direct attachment of sensors to the body can cause discomfort during sleep and make the user consciously aware of the device, potentially disturbing their sleep.

Another approach involves detecting movements during sleep by recording sound or using a camera. The sound-recording method can estimate the user's sleep state by analyzing snoring and breathing patterns, but it is easily affected by ambient noise, which can reduce accuracy. Similarly, camera-based methods can infer sleep states by detecting the user's movements through video data. However, these methods raise significant privacy concerns, and under low-light conditions, image quality deteriorates, thereby making accurate analysis difficult.

These conventional sleep monitoring methods have several problems. First, the discomfort caused by physical contact and the need to attach sensors to the body can reduce sleep quality by making the user feel uneasy during sleep. Also, the sound-recording and camera-based methods raise considerable privacy concerns, thereby causing the user to feel reluctant to use them. In particular, the camera-based methods can induce a high degree of sensitivity or anxiety in the user. Moreover, sound-based methods are easily affected by ambient noise due to the influence of external environment factors, and camera-based methods are sensitive to lighting conditions, which makes it difficult to measure sleep states accurately. Since these methods are influenced by various external factors, maintaining consistent accuracy remains challenging.

Therefore, there is a growing need for a technology that can monitor sleep states stably and accurately while minimizing user discomfort during sleep and suppressing the exposure of personal information. Furthermore, there is an increasing need for a technology capable of comprehensively evaluating sleep quality and diagnosing sleep disorders by providing information on each sleep stage.

### SUMMARY

In view of the foregoing, the present disclosure is conceived to provide a sleep stage classification device capable of accurately and reliably deriving sleep stage information of a user based on radar signals.

Also, the present disclosure is conceived to provide a sleep stage classification device capable of effectively analyzing biosignals for each sleep stage without attaching sensors to a user's body and accurately classifying the user's sleep stages.

Further, the present disclosure is conceived to provide a sleep stage classification device capable of achieving stable and consistent sleep stage analysis by minimizing the influence of external environmental factors, such as ambient noise and lighting.

Furthermore, the present disclosure is conceived to provide a sleep stage classification device capable of comprehensively monitoring a user's sleep state by evaluating sleep quality and the duration of each stage through analysis of each sleep stage.

The problems to be solved by the present disclosure are not limited to the above-described problems. There may be other problems to be solved by the present disclosure.

An aspect of the present disclosure provides a sleep stage classification device using radar signals, including: a transceiver that transmits a radar signal toward a subject and receives a radar signal reflected from the subject; and a sleep stage information generation unit that generates sleep stage information of the subject by inputting the radar signal reflected from the subject into a sleep stage classification model. The sleep stage classification model includes: a preprocessing unit that preprocesses the input radar signal; and a sleep stage classification unit that classifies sleep stages of the subject based on the preprocessed radar signal.

The above-described aspect is provided by way of illustration only and should not be construed as liming the present disclosure. Besides the above-described embodiments, there may be additional embodiments described in the accompanying drawings and the detailed description.

According to an embodiment of the present disclosure, it is possible to accurately and reliably derive sleep stage information of a user based on radar signals.

Further, it is possible to effectively analyze biosignals for each sleep stage without attaching sensors to a user's body and accurately classifying the user's sleep stages.

Furthermore, it is possible to achieve stable and consistent sleep stage analysis by minimizing the influence of external environmental factors, such as ambient noise and lighting.

Moreover, it is possible to comprehensively monitor a user's sleep state by evaluating sleep quality and the duration of each stage through analysis of each sleep stage.

## BRIEF DESCRIPTION OF THE DRAWINGS

In the detailed description that follows, embodiments are described as illustrations only since various changes and modifications will become apparent to a person with ordinary skill in the art from the following detailed description. The use of the same reference numbers in different figures indicates similar or identical items.

FIG. 1 is a configuration diagram of a sleep stage classification system according to an embodiment of the present disclosure.

FIG. 2 is a configuration diagram of a sleep stage classification device according to an embodiment of the present disclosure.

FIG. 3 is a configuration diagram of a sleep stage classification model according to an embodiment of the present disclosure.

FIG. 4 is a graph illustrating sleep stages over an eight-hour sleep cycle according to an embodiment of the present disclosure.

FIG. 5 provides graphs each illustrating an example waveform of a digital radar signal according to an embodiment of the present disclosure.

FIG. 6 is a diagram illustrating an operation of a sleep stage classification unit of the sleep stage classification model according to an embodiment of the present disclosure.

FIG. 7 shows performance evaluation results of the sleep stage classification device according to an embodiment of the present disclosure.

FIG. 8 provides graphs comparing ground-truth values and predicted values of the sleep stage classification model according to an embodiment of the present disclosure.

FIG. 9 is a graph illustrating variations in radar signals at each sleep stage according to an embodiment of the present disclosure.

FIG. 10 to FIG. 12 are flowcharts of a sleep stage classification method according to an embodiment of the present disclosure.

## DETAILED DESCRIPTION

Hereinafter, embodiments of the present disclosure will be described in detail with reference to the accompanying drawings to be readily implemented by a person with ordinary skill in the art to which the present disclosure belongs. However, it is to be noted that the present disclosure is not limited to the example embodiments but can be embodied in various other ways. In the drawings, parts irrelevant to the description are omitted in order to clearly explain the present disclosure, and like reference numerals denote like parts through the whole document.

Throughout this document, the term "connected to" may be used to designate a connection or coupling of one element to another element and includes both an element being "directly connected" another element and an element being "electronically connected" to another element via another element. Further, it is to be understood that the term "comprises or includes" and/or "comprising or including" used in the document means that one or more other components, steps, operation and/or the existence or addition of elements are not excluded from the described components, steps, operation and/or elements unless context dictates otherwise; and is not intended to preclude the possibility that one or more other features, numbers, steps, operations, components, parts, or combinations thereof may exist or may be added.

Throughout this document, the term "unit" includes a unit implemented by hardware and/or a unit implemented by software. As examples only, one unit may be implemented by two or more pieces of hardware or two or more units may be implemented by one piece of hardware.

In the present specification, some of operations or functions described as being performed by a device may be performed by a server connected to the device. Likewise, some of operations or functions described as being performed by a server may be performed by a device connected to the server.

The functionality of the elements disclosed herein may be implemented using circuitry or processing circuitry which includes general purpose processors, special purpose processors, integrated circuits, ASICs ("Application Specific Integrated Circuits"), conventional circuitry and/or combinations thereof which are configured or programmed to perform the disclosed functionality. Processors are considered processing circuitry or circuitry as they include transistors and other circuitry therein.

In the disclosure, the circuitry, units, or means are hardware that carry out or are programmed to perform the recited functionality. The hardware may be any hardware disclosed herein or otherwise known which is programmed or configured to carry out the recited functionality.

When the hardware is a processor which may be considered a type of circuitry, the circuitry, means, or units are a combination of hardware and software, the software being used to configure the hardware and/or processor.

Hereafter, embodiments of the present disclosure will be explained in detail with reference to the accompanying drawings.

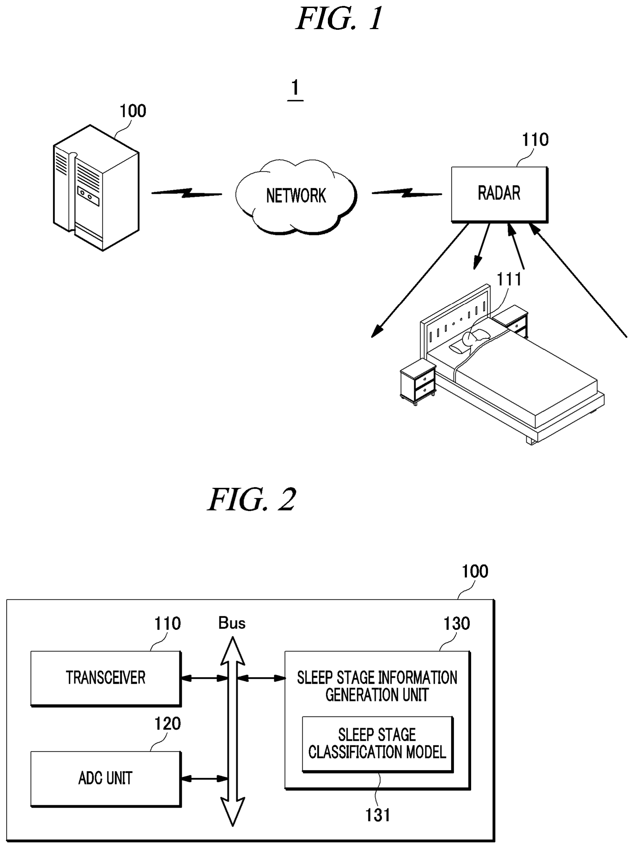

**FIG. 1** is a configuration diagram of a sleep stage classification system according to an embodiment of the present disclosure.

Referring to **FIG. 1,** a sleep stage classification system 1 may include a sleep stage classification device 100 and a radar 200.

The components of the sleep stage classification system 1 illustrated in **FIG. 1** are typically connected to each other via a network. For example, as illustrated in **FIG. 1,** the sleep stage classification system 1 and the radar 200 may be connected simultaneously or sequentially.

The network refers to a connection structure that enables information exchange between nodes such as devices, devices, etc. and includes LAN (Local Area Network), WAN (Wide Area Network), Internet (WWW: World Wide Web), a wired or wireless data communication network, a telecommunication network, a wired or wireless television network, and the like. Examples of the wireless data communication network may include 3G, 4G, 5G, 3GPP (3rd Generation Partnership Project), LTE (Long Term Evolution), WIMAX (World Interoperability for Microwave Access), Wi-Fi, Bluetooth communication, infrared communication, ultrasonic communication, VLC (Visible Light Communication), LiFi, and the like, but may not be limited thereto.

The sleep stage classification device 100 may analyze radar signals reflected from a subject 201 by using the radar 200. The sleep stage classification device 100 may detect breathing of the subject 201 during sleep based on radar signals that vary when the subject 201 is sleeping in an indoor space (for example, a bedroom). For example, the sleep stage classification device 100 may be disposed near the subject during sleep or on the ceiling of the indoor space where the subject 201 is located, and may transmit radar signals toward the subject 201 through the radar 200 and receive radar signals reflected from the subject 201. To this end, the installation direction of the radar 200 may be determined to ensure that the sleep stage classification device 100 can smoothly transmit and receive radar signals toward and from the subject 201.

The sleep stage classification device 100 may detect movements of the abdomen of the subject 201 during sleep by using the radar 200.

Accordingly, the sleep stage classification device 100 may be used to generate sleep stage information and classify sleep stages by processing radar signals even if the subject 201 does not carry or wear a separate device for detecting breathing or movements during sleep.

Herein, the sleep stage information may refer to information on sleep stages maintained during a sleep period of the subject, including details such as the start time, end time, and duration of each sleep stage.

The sleep stage classification device 100 may be implemented in a configuration in which the radar 200 is mounted on or embedded within the device so as to detect movements of the subject 201 during sleep. Further, at least some components of the sleep stage classification device 100 may be disposed in a space separate from the radar 200 and may detect movements of the subject 201 during sleep by communicating with the radar 200 through a wired or wireless network. In the following description, a transceiver of the sleep stage classification device 100 may correspond to the radar 200 illustrated in **FIG. 1.**

Hereafter, each component of the sleep stage classification device 100 will be described.

**FIG. 2** is a configuration diagram of the sleep stage classification device 100 according to an embodiment of the present disclosure.

Referring to **FIG. 2,** the sleep stage classification device 100 may include a transceiver 110, an ADC (Analog-to-Digital Converter) unit 120, and a sleep stage information generation unit 130. However, the components 110 and 120 are merely illustrative examples of components that may be controlled by the sleep stage classification device 100.

The transceiver 110 may transmit radar signals toward a subject and receive radar signals reflected from the subject.

The ADC unit 120 may convert the radar signals reflected from the subject into digital radar signals.

The sleep stage information generation unit 130 may generate sleep stage information of the subject by inputting the radar signals reflected from the subject into the sleep stage classification model 131. More specifically, the sleep stage information generation unit 130 may generate sleep stage information based on digital radar signals converted from the radar signals by the ADC unit 120.

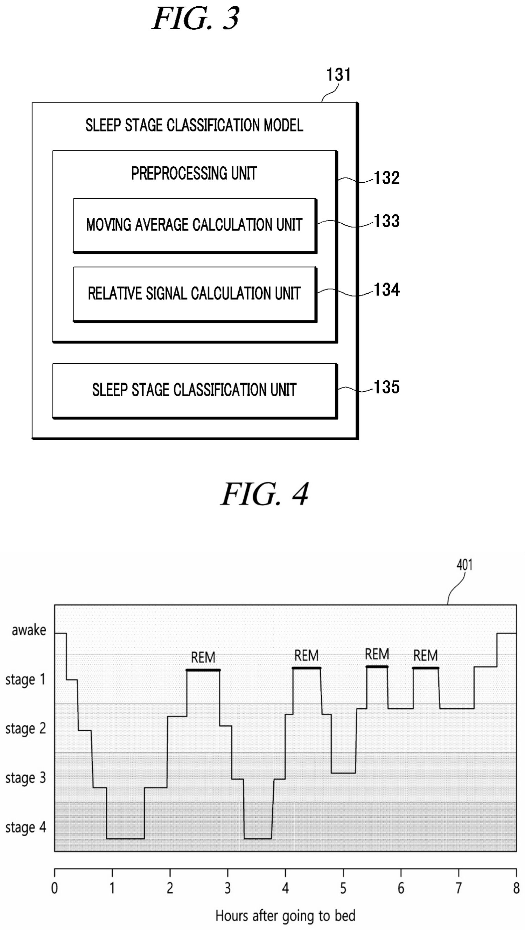

**FIG. 3** is a configuration diagram of the sleep stage classification model 131 according to an embodiment of the present disclosure.

Referring to **FIG. 3,** the sleep stage classification model 131 may include a preprocessing unit 132 and a sleep stage classification unit 135. The preprocessing unit 132 may include a moving average calculation unit 133 and a relative signal calculation unit 134.

The preprocessing unit 132 may preprocess the input radar signals. The sleep stage classification unit 135 may classify sleep stages of the subject based on the preprocessed radar signals.

The sleep stage classification model 131 may receive radar signals reflected from the subject as training data and may be trained to output the sleep stage information based on the radar signals.

The preprocessing unit 132 may include the moving average calculation unit 133 configured to calculate a moving average value for a current index of the digital radar signals, and the relative signal calculation unit 134 configured to calculate a relative signal value based on an original signal value for the current index, which is derived from the digital radar signals, and the moving average value for the current index.

Herein, the moving average calculation unit 133 may derive the moving average value for the current index to have a magnitude of at least one or greater. Also, the moving average calculation unit 133 may calculate the moving average value by averaging original signal values within a range corresponding to a predetermined window size from the current index.

Further, the relative signal calculation unit 134 may set the relative signal value for the current index to zero when the current index is smaller than the window size. Furthermore, the relative signal calculation unit 134 may calculate the relative signal value by dividing the original signal value for the current index by the moving average value for the current index.

The sleep stage classification unit 135 may generate multi-resolution radar signals by transforming normalized radar signals into a plurality of resolutions, extract feature values from the multi-resolution radar signals, and classify sleep stages of the subject based on the extracted feature values.

Herein, the sleep stages may include a wake stage, a rapid eye movement (REM) sleep stage, and a non-rapid eye movement (NREM) sleep stage.

The transceiver 110, the ADC unit 120, and the sleep stage information generation unit 130 may be program modules in the form of an operating system, application program modules, or other program modules stored in a memory module.

These program modules may include a routine, a sub-routine, a program, an object, a component, and a data architecture, each of which executes a specific task to be described later or specific abstract data, but the present disclosure is not limited thereto.

**FIG. 4** is a graph illustrating sleep stages over an eight-hour sleep cycle according to an embodiment of the present disclosure.

Referring to **FIG. 4,** it can be seen that a person's sleep stages change over time during an eight-hour sleep cycle, forming a specific pattern 401. The sleep cycle may be roughly divided into a wake stage, an NREM sleep stage, and an REM sleep stage. The wake stage generally occurs at the beginning and end of a sleep period and indicates a state before the subject falls asleep and after the subject wakes up.

When sleep begins, the subject may gradually transition into a deeper NREM sleep stage. The NREM sleep stage is further divided into stages that progress from a lighter stage to a deeper stage according to the depth of sleep. In this stage, the body relaxes, and stabilization of heart rate and breathing facilitates physical recovery, which is critical for bodily restoration.

The REM sleep stage intermittently appears during the sleep cycle. It is known as the dreaming stage and alternates with the NREM sleep stage, repeating in cycles of approximately 90 minutes. In **FIG. 4,** the REM sleep stages are represented by red bars and occur several times during sleep. In the REM sleep stage, the body's muscles are temporarily paralyzed, the eyes move rapidly, and brainwave activity becomes active.

As illustrated in **FIG. 4,** the NREM and REM sleep stages alternately repeat to form the sleep cycle, which plays a crucial role in physical recovery and mental stability.

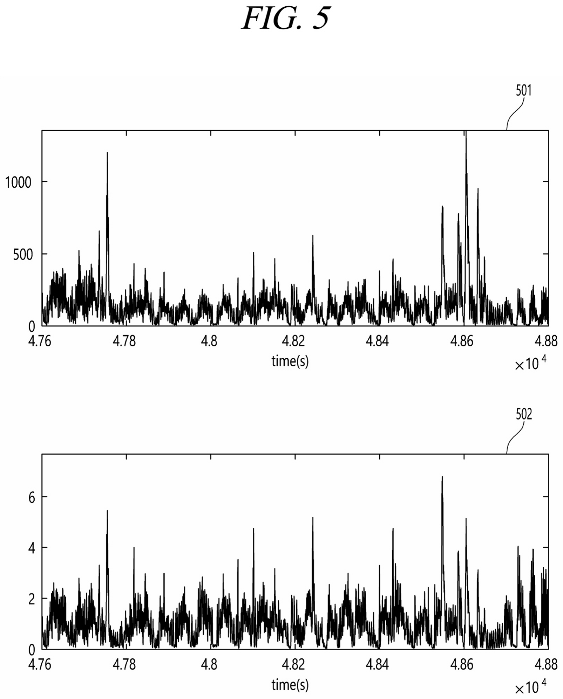

**FIG. 5** provides graphs each illustrating an example waveform of a digital radar signal according to an embodiment of the present disclosure.

Referring to **FIG. 5,** an upper graph 501 represents a time-domain waveform of digital radar signals converted by the ADC unit, and a lower graph 502 represents a waveform obtained after the digital radar signals have been normalized by the preprocessing unit.

The ADC unit functions to convert input radar signals from analog form into digital form. To this end, the ADC unit uses an equation that converts analog values into digital values based on the signal power. In the present embodiment, the ADC unit converts radar signals on a dB scale into digital values according to an equation, such as ADC = $10^{(DB/10)}$. This equation is designed to accurately express radar signal magnitude information in digital form.

The ADC unit generally uses an ADC chip capable of fast sampling to facilitate high-speed signal conversion. For example, in environments where high-speed data acquisition is required, a high-performance ADC chip may be used. Such an ADC chip provides both high-speed conversion and high resolution, thereby enabling precise conversion of analog radar signals into digital radar signals.

Accordingly, the upper graph 501 of **FIG. 5** illustrates a waveform of analog signals which are collected by the radar and converted into digital signals by the ADC unit, and the lower graph 502 illustrates a stable waveform obtained after the digital signals are normalized by the preprocessing unit.

In the present disclosure, preprocessing of radar signals may be performed within the sleep stage classification model. Conventionally, input data for a deep learning model were prepared through separate preprocessing before radar signals were input into the model. However, the preprocessing executed by the CPU consumes considerable time, which may reduce overall computational efficiency.

In the present disclosure, to solve these problems, the functions of the preprocessing unit are incorporated into the sleep stage classification model of the sleep stage classification unit. The preprocessing of the sleep stage classification model is designed to be performed within the deep learning model. Accordingly, instead of preprocessing the digital radar signals before they are input into the deep learning model, the digital radar signals are directly input into the model and the preprocessing is then performed as one of the deep learning layers.

This configuration allows the preprocessing to be executed in parallel by the GPU, thereby significantly improving computational efficiency compared to conventional CPU-based methods. By integrating the preprocessing unit into the deep learning model, the total computation time is reduced and the computational load is decreased, thereby enabling faster and more efficient sleep stage classification.

Hereafter, an operation of the preprocessing unit will be described in more detail.

The preprocessing unit may perform a normalization process based on a moving average for the digital radar signals to stabilize the input data. The preprocessing unit may be composed of the moving average calculation unit and the relative signal calculation unit. A detailed operation of each component will be described as follows.

The moving average calculation unit may calculate a moving average value over a specific range corresponding to the current index of the digital radar signals.

<Equation 1>

$$\text{meanVal}(i) = \max\left(\frac{1}{\text{wSizeMin}} \sum_{j=i-\text{wSizeMin}+1}^{i} \text{signal\_rad}(j), 1\right)$$

In Equation 1, a moving average value (meanVal(i)) may be obtained by averaging original signal values (signal_rad(j)) included within a range corresponding to a predetermined window size (wSizeMin) from a current index (i). Herein, the original signal values may refer to digital radar signals converted by the ADC unit.

Accordingly, the moving average calculation unit may calculate an average value that reflects the overall signal magnitude within the current range and designate it as the moving average value meanVal(i). Further, to ensure stable normalization, a minimum value may be set to 1 when the moving average value is less than 1, thereby keeping the moving average value from becoming excessively small.

Subsequently, the relative signal calculation unit may calculate a relative signal value of the digital radar signals by using the moving average value calculated by the moving average calculation unit.

<Equation 2>

$$\text{signal\_rad\_rel}(i) = \frac{\text{signal\_rad}(i)}{\text{meanVal}(i)}$$

In Equation 2, a relative signal value (signal_rad_rel(i)) may be defined as the value obtained by dividing the original signal value (signal_rad(i)) for the current index by the moving average value (meanVal(i)). Accordingly, each signal value represents its relative magnitude with respect to an average magnitude at that point in time, thereby reducing deviations in the input data and improving stability.

In particular, the relative signal calculation unit may set the relative signal value to zero when the current index is smaller than the window size, thereby suppressing the occurrence of errors in an initial range in which sufficient data has not yet been accumulated.

In the present disclosure, through the above-described operation of the preprocessing unit, it is possible to stably calculate relative signal magnitudes by normalizing original signal values based on the moving average. Thus, it is possible to provide input data optimized for training the deep learning model.

In the present disclosure, by incorporating the time-consuming preprocessing into the deep learning model and enabling parallel computation on the GPU, the total computation time can be significantly reduced. To verify this, tests were conducted to compare cases where the preprocessing was incorporated into the sleep stage classification model and cases where the preprocessing was not incorporated.

The test was conducted using one day's sleep data, collected at a frequency of 5 Hz with a batch size of 1,440. The test environment consisted of an AMD Threadripper 5975WX CPU, 512 GB RAM, and an NVIDIA GeForce RTX 4090 GPU.

As a result, when the preprocessing was separated from the model, an average execution time was 580.1 ms, whereas when the preprocessing was integrated into the model and executed on the GPU, the average execution time was significantly reduced to 42.3 ms. Further, when the preprocessing was separated from the model, a standard deviation was 12.2 ms, whereas when the preprocessing layer was incorporated into the model, it was decreased to 0.3 ms, thereby confirming that the stability of computation time was also improved.

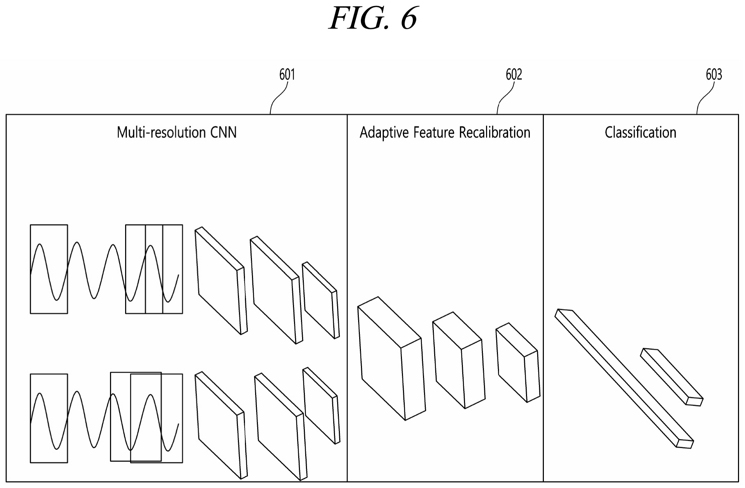

FIG. 6 is a diagram illustrating an operation of a sleep stage classification unit of the sleep stage classification model according to an embodiment of the present disclosure.

Referring to FIG. 6, the sleep stage classification unit may generate sleep stage information based on a multi-resolution CNN transformation stage 601, an adaptive feature recalibration stage 602, and a classification stage 603.

The relative signal value generated by the preprocessing unit described above may be input into the sleep stage classification unit. That is, training data used for the sleep stage classification unit may include time-series relative signal values of the subject that are output by the preprocessing unit.

First, the stage 601 may represent a CNN (Convolutional Neural Network) structure designed to process input data at various resolutions. In this stage, original radar signal data are converted into a plurality of resolutions, and features are extracted for each resolution by allowing the data to pass through corresponding CNN layers. Accordingly, information on various sizes and shapes of the data can be captured simultaneously, and features obtained from different resolutions may be integrated in subsequent layers to enable a richer feature representation. The multi-resolution CNN provides the flexibility needed to effectively detect and analyze diverse signal variations occurring during sleep.

Then, the stage 602 may represent a technique that improves model performance by dynamically recalibrating extracted feature maps to assign higher weights to important features and suppress less significant features. In this process, both channel-wise and spatial information of the feature maps are considered during recalibration, thereby enabling the model to respond robustly to different conditions according to the input. Adaptive feature recalibration may adjust the importance of features through a gating mechanism. Herein, the gate strengthens key features or suppresses specific features as needed, thereby enabling the model to achieve more accurate and reliable sleep stage classification.

Finally, the stage 603 represents a process of classifying the final sleep stage using fully connected layers. In this stage, the extracted and recalibrated feature values are used to determine sleep stages, such as a wake stage, an REM sleep stage, and an NREM sleep stage. This classification process reflects the characteristics of each sleep stage to predict the final sleep state, thereby enabling effective analysis of a user's sleep patterns.

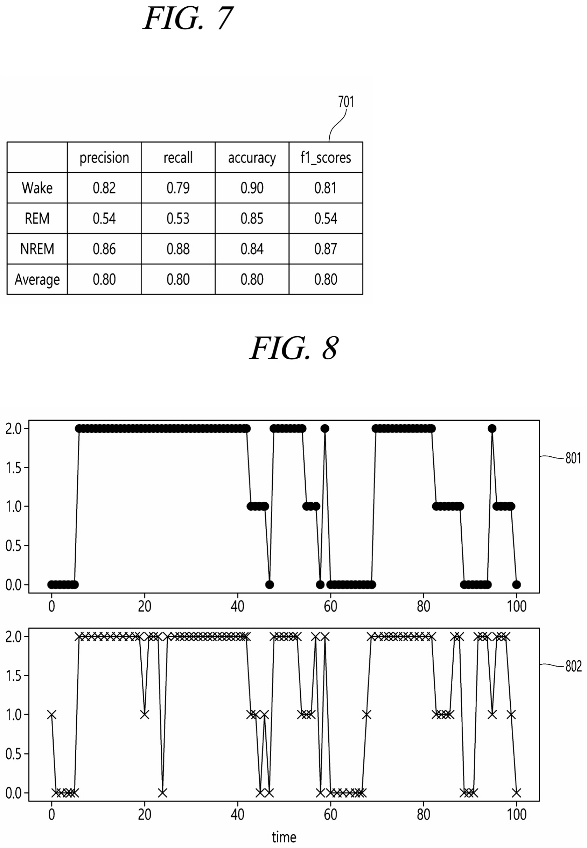

FIG. 7 shows performance evaluation results of the sleep stage classification device according to an embodiment of the present disclosure.

Referring to FIG. 7, a table shows performance evaluation results of the sleep stage classification device for each sleep stage, based on precision, recall, accuracy, and F1_scores. These evaluation results are intended to verify whether the sleep stage classification device demonstrates reliable classification performance for the wake (Wake) stage, rapid eye movement (REM) sleep, and non-rapid eye movement (NREM) sleep stage.

In the wake stage, the precision is 0.82, the recall is 0.79, the accuracy is 0.90, and the F1_score is 0.81, which indicates relatively high performance. This suggests that the sleep stage classification device can accurately predict sleep stages during wakefulness.

In the REM sleep stage, the precision is 0.54, the recall is 0.53, the accuracy is 0.85, and the F1_score is 0.54, which indicates relatively low performance. This suggests that the REM sleep stage may have more complex and variable features than the other sleep stages, which may result in relatively low classification performance.

In the NREM sleep stage, the precision is 0.86, the recall is 0.88, the accuracy is 0.84, and the F1_score is 0.87, which indicates high performance. This demonstrates that the sleep stage classification device is capable of stably recognizing and classifying the NREM sleep stage.

On average, the precision, recall, accuracy, and F1_score across all sleep stages are each 0.80, which confirms that the sleep stage classification device exhibits overall stable and reliable performance.

**FIG. 8** provides graphs comparing ground-truth values and predicted values of the sleep stage classification model according to an embodiment of the present disclosure.

Referring to **FIG. 8,** an upper graph 801 represents actual ground-truth values (Target) for sleep stage classification, which include NREM, REM, and WAKE states. A lower graph 802 represents sleep stage results predicted by the sleep stage classification model, in which predicted values for sleep stages are displayed over time. Accordingly, **FIG. 8** allows the verification of the agreement between the actual sleep stages and the model's predictions.

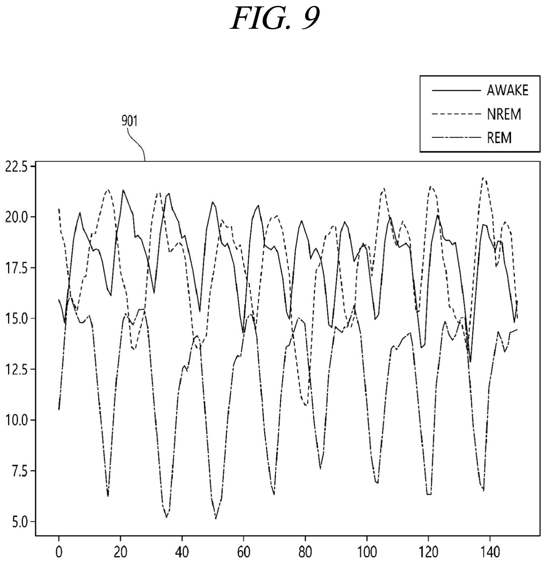

**FIG. 9** is a graph illustrating variations in radar signals at each sleep stage according to an embodiment of the present disclosure.

A graph 901 in **FIG. 9** illustrates variations in radar signals over time during the wake stage (AWAKE), non-rapid eye movement sleep stage (NREM), and rapid eye movement sleep stage (REM). As shown in the graph, the radar signal patterns differ across sleep stages, which enables the characteristics of radar signals for each sleep stage to be distinguished.

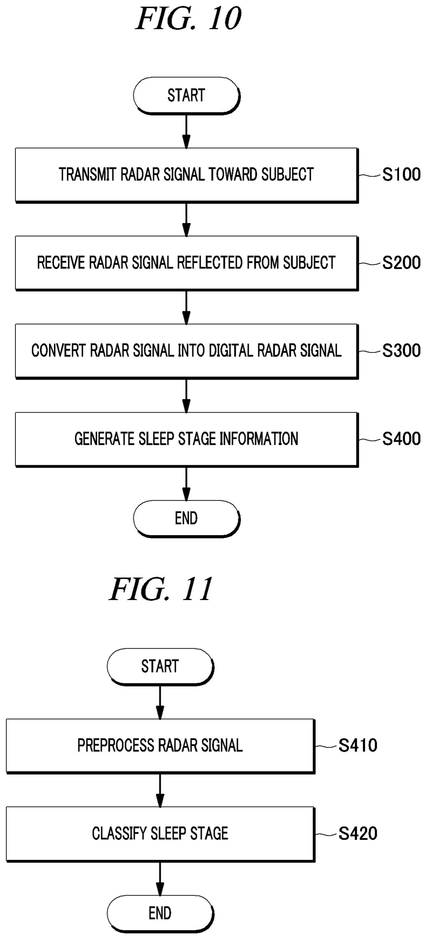

**FIG. 10** to **FIG. 12** are flowcharts of a sleep stage classification method according to an embodiment of the present disclosure.

The sleep stage classification method illustrated in **FIG. 10** to **FIG. 12** includes processes that are sequentially performed according to the embodiments shown in **FIG. 1** to **FIG. 9**. Accordingly, the omitted details can be found by referring to the sleep stage classification method performed by the sleep stage classification device according to the embodiments shown in **FIG. 1** to **FIG. 9.**

Referring to **FIG. 10,** the sleep stage classification method is performed by the sleep stage classification device, and may include a process S100 of transmitting a radar signal toward a subject, a process S200 of receiving a radar signal reflected from the subject, a process S300 of converting the radar signal into a digital radar signal, and a process S400 of generating sleep stage information.

Herein, the process S400 of generating sleep stage information may include a process of inputting digital radar signals into the sleep stage classification model.

**FIG. 11** is a flowchart illustrating operations of the sleep stage classification model according to an embodiment of the present disclosure. The sleep stage classification model may operate through a process S410 of preprocessing the input radar signal and a process S420 of classifying sleep stages based on the preprocessed radar signal.

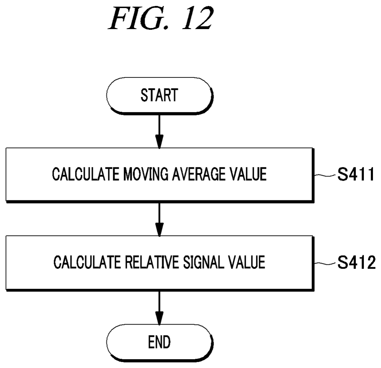

**FIG. 12** is a flowchart illustrating a process of preprocessing radar signals according to an embodiment of the present disclosure. The process of preprocessing radar signals may include a process S411 of calculating a moving average value for a current index of the digital radar signals, and a process S412 of calculating a relative signal value based on an original signal value for the current index, which is derived from the digital radar signals, and the moving average value for the current index.

The process S411 of calculating a moving average value may include a process of deriving the moving average value for the current index to have a magnitude of at least one or greater.

The process S411 of calculating a moving average value may also include a process of calculating the moving average value by averaging original signal values within a range corresponding to a predetermined window size from the current index.

The process S412 of calculating a relative signal value may include a process of setting the relative signal value for the current index to zero when the current index is smaller than the window size.

The process S412 of calculating a relative signal value may further include a process of calculating the relative signal value by dividing the original signal value for the current index by the moving average value for the current index.

The method for classifying a sleep stage described above with reference to FIG. 1 to FIG. 12 can be implemented in a computer program stored in a medium to be executed by a computer or a storage medium including instructions codes executable by a computer. Also, the method for for classifying a sleep stage described above with reference to FIG. 1 to FIG. 12 can be implemented in a computer program stored in a medium to be executed by a computer.

A computer-readable medium can be any usable medium which can be accessed by the computer and includes all volatile/non-volatile and removable/non-removable media. Further, the computer-readable medium may include all computer storage and communication media. The computer storage medium includes all volatile/non-volatile and removable/non-removable media embodied by a certain method or technology for storing information such as computer-readable instruction code, a data structure, a program module or other data. The communication medium typically includes the computer-readable instruction code, the data structure, the program module, or other data of a modulated data signal such as a carrier wave, or other transmission mechanism, and includes a certain information transmission medium.

The method may be further divided into additional steps or combined into fewer steps through the embodiments described above with reference to FIGS. 1 to 12. In addition, some steps may be omitted as necessary, and the order of the steps may be changed.

The above description of the present disclosure is provided for the purpose of illustration, and it would be understood by those skilled in the art that various changes and modifications may be made without changing technical conception and essential features of the present disclosure. Thus, it is clear that the above-described embodiments are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

Explanation of codes

    100: Sleep stage classification device
    200: Radar
    201: Subject

**Claims**

1.  A sleep stage classification device using radar signals, comprising:

    a transceiver configured to transmit a radar signal toward a subject and receive a radar signal reflected from the subject; and
    a sleep stage information generation unit configured to generate sleep stage information of the subject by inputting the radar signal reflected from the subject into a sleep stage classification model,
    wherein the sleep stage classification model includes:

        a preprocessing unit configured to preprocess the input radar signal; and
        a sleep stage classification unit configured to classify a sleep stage of the subject based on the preprocessed radar signal.

2.  The sleep stage classification device of Claim 1,
    wherein the sleep stage classification model is configured to:

    receive the radar signal reflected from the subject as training data; and
    be trained to output the sleep stage information based on the radar signal.

3.  The sleep stage classification device of Claim 1, further comprising:

    an ADC (Analog-to-Digital Converter) unit configured to convert the input radar signal into a digital radar signal,
    wherein the sleep stage information generation unit is further configured to input the digital radar signal into the sleep stage classification model.

4.  The sleep stage classification device of Claim 3,
    wherein the preprocessing unit includes:

a moving average calculation unit configured to calculate a moving average value for a current index of the digital radar signal; and

a relative signal calculation unit configured to calculate a relative signal value based on an original signal value for the current index, which is derived from the digital radar signal, and the moving average value for the current index.

5. The sleep stage classification device of Claim 4,
wherein the moving average calculation unit is further configured to derive the moving average value for the current index to have a magnitude of at least one or greater.

6. The sleep stage classification device of Claim 4,
wherein the moving average calculation unit is further configured to calculate the moving average value by averaging original signal values within a range corresponding to a predetermined window size from the current index.

7. The sleep stage classification device of Claim 6,
wherein the relative signal calculation unit is further configured to set the relative signal value for the current index to zero when the current index is smaller than the window size.

8. The sleep stage classification device of Claim 4,
wherein the relative signal calculation unit is further configured to calculate the relative signal value by dividing the original signal value for the current index by the moving average value for the current index.

9. The sleep stage classification device of Claim 4,
wherein the sleep stage classification unit is further configured to generate the sleep stage information by classifying the sleep stage of the subject for each time point based on the relative signal value.

10. The sleep stage classification device of Claim 1,
wherein the sleep stage classification unit is further configured to:

generate a multi-resolution radar signal by transforming the preprocessed radar signal into a plurality of resolutions;
extract a feature value from the multi-resolution radar signal; and
classify the sleep stage of the subject based on the extracted feature value.

11. The sleep stage classification device of Claim 1,
wherein the sleep stages include a wake stage, a rapid eye movement (REM) sleep stage, and a non-rapid eye movement (NREM) sleep stage.

12. A sleep stage classification method that is performed by a sleep stage classification device using radar signals, comprising:

transmitting a radar signal toward a subject;
receiving a radar signal reflected from the subject; and
generating sleep stage information of the subject by inputting the radar signal reflected from the subject into a sleep stage classification model,
wherein the sleep stage classification model is configured to operate through a process of preprocessing the input radar signal and a process of classifying sleep stages based on the preprocessed radar signal.

13. The sleep stage classification method of Claim 12,
wherein the sleep stage classification model is further configured to:

receive the radar signal reflected from the subject as training data and
be trained to output the sleep stage information based on the radar signal.

14. The sleep stage classification method of Claim 12, further comprising:

converting the input radar signal into a digital radar signal,
wherein the generating sleep stage information includes a process of inputting the digital radar signal into the sleep stage classification model.

15. The sleep stage classification method of Claim 14,
wherein the preprocessing includes:

calculating a moving average value for a current index of the digital radar signal; and
calculating a relative signal value based on an original signal value for the current index, which is derived from the digital radar signal, and the moving average value for the current index.

## FIG. 1

## FIG. 2

# FIG. 3

# FIG. 4

*FIG. 5*

## FIG. 6

## FIG. 7

| | precision | recall | accuracy | f1_scores |
|---|---|---|---|---|
| Wake | 0.82 | 0.79 | 0.90 | 0.81 |
| REM | 0.54 | 0.53 | 0.85 | 0.54 |
| NREM | 0.86 | 0.88 | 0.84 | 0.87 |
| Average | 0.80 | 0.80 | 0.80 | 0.80 |

## FIG. 8

# FIG. 9

# FIG. 10

```
                        ┌─────────┐
                        │  START  │
                        └─────────┘
                             │
                             ▼
        ┌────────────────────────────────────────────┐
        │    TRANSMIT RADAR SIGNAL TOWARD SUBJECT     │───S100
        └────────────────────────────────────────────┘
                             │
                             ▼
        ┌────────────────────────────────────────────┐
        │   RECEIVE RADAR SIGNAL REFLECTED FROM SUBJECT│──S200
        └────────────────────────────────────────────┘
                             │
                             ▼
        ┌────────────────────────────────────────────┐
        │  CONVERT RADAR SIGNAL INTO DIGITAL RADAR SIGNAL│─S300
        └────────────────────────────────────────────┘
                             │
                             ▼
        ┌────────────────────────────────────────────┐
        │       GENERATE SLEEP STAGE INFORMATION      │───S400
        └────────────────────────────────────────────┘
                             │
                             ▼
                        ┌─────────┐
                        │   END   │
                        └─────────┘
```

# FIG. 11

```
                        ┌─────────┐
                        │  START  │
                        └─────────┘
                             │
                             ▼
        ┌────────────────────────────────────────────┐
        │           PREPROCESS RADAR SIGNAL           │───S410
        └────────────────────────────────────────────┘
                             │
                             ▼
        ┌────────────────────────────────────────────┐
        │             CLASSIFY SLEEP STAGE            │───S420
        └────────────────────────────────────────────┘
                             │
                             ▼
                        ┌─────────┐
                        │   END   │
                        └─────────┘
```

*FIG. 12*

```
         ┌──────────────┐
         │    START     │
         └──────┬───────┘
                │
                ▼
 ┌───────────────────────────────────┐
 │  CALCULATE MOVING AVERAGE VALUE    │──── S411
 └───────────────┬───────────────────┘
                 │
                 ▼
 ┌───────────────────────────────────┐
 │  CALCULATE RELATIVE SIGNAL VALUE   │──── S412
 └───────────────┬───────────────────┘
                 │
                 ▼
         ┌──────────────┐
         │     END      │
         └──────────────┘
```

EUROPEAN SEARCH REPORT

Application Number

EP 25 22 2900

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TOFTEN STÅLE ET AL: "Validation of sleep stage classification using non-contact radar technology and machine learning (Somnofy )", SLEEP MEDICINE ELSEVIER, AMSTERDAM, NL, vol. 75, 6 March 2020 (2020-03-06), pages 54-61, XP086343007, ISSN: 1389-9457, DOI: 10.1016/J.SLEEP.2020.02.022 [retrieved on 2020-03-06] * pages 1-4 * | 1-15 | INV. A61B5/00 A61B5/0507 |
| X | KWON HYUN BIN ET AL: "Attention-Based LSTM for Non-Contact Sleep Stage Classification Using IR-UWB Radar", IEEE JOURNAL OF BIOMEDICAL AND HEALTH INFORMATICS IEEE, PISCATAWAY, NJ, USA, vol. 25, no. 10, 13 April 2021 (2021-04-13), pages 3844-3853, XP011881370, ISSN: 2168-2194, DOI: 10.1109/JBHI.2021.3072644 [retrieved on 2021-10-04] * pages 1-4, figure 2 * | 1-15 | |
| A | LU YI ET AL: "Sleep stage estimation method based on state transition using millimeter-wave radar", 20240328, vol. 13091, 28 March 2024 (2024-03-28), pages 130911A-130911A, XP060201857, ISSN: 0277-786X, DOI: 10.1117/12.3023191 ISBN: 9781510675056 * the whole document * | 1-15 | |

**TECHNICAL FIELDS SEARCHED (IPC)**

A61B

The present search report has been drawn up for all claims

| Place of search | Date of completion of the search | Examiner |
|---|---|---|
| The Hague | 29 April 2026 | Horrix, Doerte |

EPO FORM 1503 03.82 (P04C01)

**EP 4 781 897 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020250010059